(19) Europäisches Patentamt
European Patent Office
Office européen des brevets

(11) **EP 1 217 984 B1**

(12) **EUROPÄISCHE PATENTSCHRIFT**

(45) Veröffentlichungstag und Bekanntmachung des Hinweises auf die Patenterteilung:
**22.12.2004 Patentblatt 2004/52**

(21) Anmeldenummer: **99900054.0**

(22) Anmeldetag: **08.10.1999**

(51) Int Cl.⁷: **A61K 7/42**, A61K 7/48, A61K 7/00

(86) Internationale Anmeldenummer:
**PCT/DE1999/003295**

(87) Internationale Veröffentlichungsnummer:
**WO 2001/026617 (19.04.2001 Gazette 2001/16)**

(54) **KOSMETISCHE WIRKSTOFFZUBEREITUNG MIT SYNERGISTISCH ERHÖHTEM RADIKALSCHUTZFAKTOR**

COSMETIC PREPARATION OF ACTIVE SUBSTANCES WITH A SYNERGISTICALLY INCREASED RADICAL PROTECTION FACTOR

PREPARATION COSMETIQUE A BASE DE PRINCIPES ACTIFS PRESENTANT UN FACTEUR DE PROTECTION ANTI-RADICAUX LIBRES SYNERGIQUEMENT ELEVE

(84) Benannte Vertragsstaaten:
**AT BE CH CY DE DK ES FI FR GB GR IE IT LI LU MC NL PT SE**

(43) Veröffentlichungstag der Anmeldung:
**03.07.2002 Patentblatt 2002/27**

(73) Patentinhaber: **Coty B.V.**
**2031 CC Haarlem (NL)**

(72) Erfinder:
• **GOLZ-BERNER, Karin**
**MC-98000 Monaco (MC)**
• **ZASTROW, Leonhard**
**MC-98000 Monaco (MC)**

(74) Vertreter: **Walter, Wolf-Jürgen et al**
**Anwaltskanzlei**
**Gulde Hengelhaupt Ziebig & Schneider**
**Wallstrasse 58/59**
**10179 Berlin (DE)**

(56) Entgegenhaltungen:
**EP-A- 1 312 373**      **WO-A-01/85182**
**FR-A- 2 672 605**      **FR-A- 2 758 984**
**FR-A- 2 762 008**      **FR-A- 2 770 228**
**GB-A- 2 237 805**

• **PATENT ABSTRACTS OF JAPAN Bd. 1999, Nr. 05 31 Mai 1999 & JP 11 035 444 A (NIKKO CHEM KK; LYCORED NATURAL PROD IND LTD) 09 Februar 1999**

**Beschreibung**

Die erfindungsgemäße kosmetische Wirkstoffzubereitung als Bestandteil kosmetischer Präparate schützt in besonders wirksamer Weise gegen den Angriff freier Radikale auf die Haut sowohl allein als auch in Kombination mit anderen Wirkstoffen.

Bekanntlich sind freie Radikale, wie Superoxid-Ionen, Hydroxy-Radikale, Oxide) für einen Gewebeabbau und damit für die Erscheinung des Alterns der Haut verantwortlich. Die Proteine und Lipide der Zellmembranen werden zerstört, die DNA beschädigt und eine Schlüsselsubstanz der Haut, Hyaluronsäure, wird ebenfalls abgebaut. Unter normalen biologischen Bedingungen gibt es ein Gleichgewichtsverhältnis zwischen auftretenden freien Radikalen und deren Eindämmung durch körpereigene chemische oder enzymatische Systeme. Durch zusätzliche Streßfaktoren von außen, wie aggressive Atmosphäre, Tabakrauch, Ultraviolettstrahlung usw. werden diese körpereigenen Abwehrsysteme überfordert und das Gleichgewicht zugunsten der freien Radikale gestört. Es treten Entzündungs- oder Alterungserscheinungen der Haut auf, und ein Ausgleich durch kosmetische Produkte ist angezeigt.

Es sind bereits eine Reihe von Produkten für diesen Zweck vorgeschlagen worden, die oftmals Vitaminmischungen mit den Vitaminen A, C und E enthalten oder Zusätze von Superoxiddismutase oder Extrakte aus bestimmten pflanzlichen oder tierischen Lebewesen. So ist aus der US-A-5,629,185 eine kosmetische Zusammensetzung bekannt, die Ultraschallaufschlußprodukte von Hefen oder anderen Zelldispersionen enthält. Weiterhin gibt es eine Vielzahl von Veröffentlichungen, in denen die Verwendung reiner Pflanzenextrakte für kosmetische Zwecke beschrieben ist, wie z.B. in der WO97/45100 mit einem Gemisch von sieben verschieden Extrakten zum Bekämpfung von Cellulite.

Die Suche nach weiteren wirksamen Stoffen ist ein wesentliches Element kosmetischer Forschung. Weiterhin problematisch bei vielen dieser Produkte ist es, daß die gegen freie Radikale wirksamen Stoffe ihr Fängerpotential innerhalb der fertigen kosmetischen Zusammensetzung oftmals nicht auf Dauer beibehalten, d.h. es sind besondere Formulierungen erforderlich, damit die Wirksamkeit der Radikalfänger auf Dauer erhalten bleibt.

Aus der US-A-5552378 und weiteren Patenten ist bekannt, Cyclodextrine auch in Kosmetika für die langsame Freisetzung der Aktivbestandteile oder zur Unterdrückung von Körpergerüchen einzusetzen.

Zum anderen scheint sich in der kosmetischen Industrie die Kenntnis bisher noch nicht durchgesetzt zu haben, daß die Möglichkeit besteht, das antioxidative Potential der Haut zu messen (DE 4328639) und neuerdings auch mit Hilfe eines relativ einfachen Verfahrens den Radikalschutzfaktor einer kosmetischen Zubereitung zu bestimmen und damit gezielt Materialien in eine solche Zubereitung einzubeziehen.

Es ist Aufgabe der vorliegenden Erfindung, eine kosmetische Wirkstoffzubereitung bereitzustellen, bei der das Radikalschutzpotential effektiv erhöht wird ohne Zugabe weiterer Wirkstoffe mit radikalfangenden Eigenschaften.

Eine weitere Aufgabe der Erfindung ist die Bereitstellung spezieller kosmetischer Zusammensetzungen, die diese Wirkstoffzubereitung enthalten und speziell solcher Wirkstoffzubereitungen, die eine weitere Verbesserung der Eigenschaften insbesondere hinsichtlich der Öffnung der Hautporen erreicht.

Erfindungsgemäß ist die kosmetische Wirkstoffzubereitung mit hohem Radikalschutzfaktor gekennzeichnet durch einen Gehalt an

(a) einem durch Extraktion der Rinde von <u>Quebracho blanco</u> und nachfolgender enzymatischer Hydrolyse gewonnenem Produkt, das wenigstens 90 Gew- % Proanthocyanidin-Oligomere und höchstens 10 Gew- % Gallussäure enthält, wobei der Gehalt von (a), das in einer an Mikrokapseln gebundenen Wirkstoffkonzentration von 2 Gew- % vorliegt, im Bereich von 0,1 bis 10 Gew- % liegt;

(b) einem durch Extraktion gewonnenen Seidenraupenextrakt, der das Peptid Cecropine, Aminosäuren und ein Vitamingemisch enthält, wobei der Gehalt von (b) im Bereich von 0,1 bis 10 Gew-% liegt;

(c) einem nichtionischen, kationischen oder anionischen Hydro-Gel oder Gemisch von Hydro-Gelen mit einem Gehalt von (c) im Bereich von 0,1 bis 5 Gew-%;

(d) einem oder mehreren Phospholipiden mit einem Anteil von 0,1 bis 30 Gew-%;

(e) einem wenigstens 150 Einheiten Superoxiddismutase pro ml enthaltenden Ultraschall-Aufschlußprodukt einer Hefe, wobei der Gehalt des Aufschlußproduktes im Bereich von 0,5 bis 4 Gew- % liegt;

(f) einem oder mehreren Cyclodextrinen, ausgewählt unter α-, β- und γ-Cyclodextrinen, mit einem Gehalt von 0,5 bis 8 Gew-%; und

(g) dem Rest Wasser;

jeweils bezogen auf das Gesamtgewicht der wirkstoffzubereitung.

jeweils bezogen auf die Gesamtmasse der Wirkstoffzubereitung.

Gegebenenfalls kann die Wirkstoffzubereitung weiterhin einen Extrakt von Acerolafrüchten <u>Malpighia punidifolia</u> enthalten, wobei dessen Gehalt im Bereich von 1-30 Gew-% liegt.

Der erfindungsgemäße Quebracho-Rindenextrakt bzw. dessen Hydrolyseprodukt enthält einen sehr hohen Anteil Proanthocyanidine, die kondensierte Tannine darstellen. Diese in Form von Oligomeren auftretenden Verbindungen sowie der geringe Anteil an Gallussäure zeigt in dieser Kombination und in Konzentrationen zwischen 1 und 10 Gew-% eine deutliche Radikalschutzwirkung, die die Wirkung von Superoxiddismutase (SOD) bei weitem übertrifft. Die Aktivität gegen freie Radikale wurde mit der von SOD verglichen und lag bei einer 1 Gew-%igen Lösung des Extraktes bei 42 % (SOD 4 %), einer 2,5 Gew-%igen Lösung bei 83 % (SOD 15 %) und einer 5 Gew-%igen Lösung bei 100 % (SOD 38 %).

Bevorzugt enthält der Extrakt (a) wenigstens 95 Gew- % Proanthocyanidin-Oligomere und höchstens 5 Gew-% Gallussäure, insbesondere wenigstens 99 Gew- % Proanthocyanidin-Oligomere und höchstens 1 Gew-% Gallussäure.

Der Gehalt von (a) beträgt 1 bis 10 Gew-%, wobei der Wirkstoff aus der Quebrachorinde in Mikrokapseln eingeschlossen ist. Die Mikrokapseln bestehen z. B. aus Petrolatum, Natriumtristearat, Agar, Phenonip und Wasser.

Der Seidenraupenextrakt (b) wird durch Extraktion der Seidenraupe (Bombyx mori) mit Propylenglycol gewonnen und enthält Vitamine, Aminosäuren und das Peptid Cecropine, das eine besondere antibakterielle Wirksamkeit aufweist. In den letzten Jahren haben eine Reihe von Untersuchungen an der Hämolymphe und der Kutikularmatrix des Seidenwurmes gezeigt, daß nicht nur antibakterielle Peptide sondern auch Inhibierungsmittel von insbesondere fungalen Proteasen darin enthalten sind. Weiterhin zeigen derartige Extrakte sauerstoffverbrauchende Eigenschaften, wodurch sie den Zellmetabolismus aktivieren, und sie haben feuchtigkeitshaltende Eigenschaften, eine deutliche Heilwirkung auf Hautläsionen durch Verkürzung der Heilungszeit sowie eine glättende Wirkung auf die Haut.

Bevorzugt enthält der Extrakt (b) die Aminosäuren Aspartinsäure, Asparagin, Threonin, Serin, Glutaminsäure, Prolin, Glycin, Alanin, Valin, Cystein, Methionin, Isoleucin, Leucin, Tyrosin, Phenylalanin, Lysin, Histidin, Arginin.

Weiterhin bevorzugt enthält der Extrakt (b) ein Vitamingemisch mit den Vitaminen $B_1$, $B_2$, $B_5$, $B_6$, $B_8$, $B_9$, $B_{12}$, PP, A, E und C.

Die Bestandteile (a) und (b) liegen vorzugsweise in Konzentrationen von jeweils 0,1 bis 3 Gew-% in der Wirkstoffzubereitung vor, insbesondere 0,5 bis 3 Gew-%.

Das erfindungsgemäß enthaltende Gel, das auch ein Gemisch verschiedener Gele sein kann, ist ein Hydrogel, das wasserlöslich ist bei Temperaturen von höher als etwa 40 bis 50 °C und die Gelstruktur bei niedrigeren Temperaturen zwischen 10 und 30 °C ausbildet. Beispiele für derartige Gele sind nichtionische Polymere wie Polyvinylalkohol, Polyvinylpyrrolidonmodifizierte Maisstärke und Hydroxyethylcellulose; kationische Polymere wie kationischer Guar, kationische Cellulose, synthetische kationische Polymere; oder Gele wie Gelatine, Carrageenan, Bentonit-Gele, Copolymer-Gele wie Carbomer.

Die erfindungsgemäß enthaltenen Phospholipide sind ausgewählt unter Phosphatidylcholin, Phosphatidylethanolamin, Phosphatidylinositol, Phosphatidylserin, Phosphatidsäure und Lysolecithine sowie Gemischen davon. Bekannte Produkte sind beispielsweise Phoslipon® . Der Anteil der Phospholipide liegt im Bereich von 0,1 bis 30 Gew-%, vorzugsweise 0,5 bis 20 Gew-%.

Die Komponenten (a) und (b) der Wirkstoffzubereitung bilden mit den Phospholipiden (d) vermutlich assoziationsähnliche Gebilde verschiedener Moleküle, die wiederum in der sich ausbildenden Struktur des Gels (c)+(g) in weitgehend gleichmäßiger Verteilung angelagert werden und die hier insgesamt als "Assoziationskomplex" bezeichnet werden. Zusammen mit den Cyclodextrinen und dem SOD-haltigen Hefeaufschlußprodukt, das selbst eine Reihe unterschiedlicher Substanzen enthält, ergibt sich nach den bisherigen Erkenntnissen ein stabiles Zusammenlagerungsgebilde innerhalb des Gels von noch nicht geklärter Struktur, bei dem der Radikalschutzfaktor (Erläuterung weiter unten) deutlich über dem summarisch zu erwartenden Wert liegt und wodurch ein Synergismus begründet wird. Die Gelstruktur scheint dabei einen bedeutenden Einfluß auf die Zusammenlagerung der verschiedenen Substanzen zu haben und ist wahrscheinlich ausschlaggebend für die Stabilität des Gebildes.

Durch Zusatz von Acerola-Extrakt sowie bestimmter Pflanzenextrakte kann eine weitere Steigerung des Radikalschutzfaktors erfolgen.

Die Wirkstoffzubereitung kann vorteilhaft neben den Grundbestandteilen (a) bis (g) weiterhin verschiedene Pflanzenextrakte enthalten, wie Citrusschalen- oder -blatt-Extrakte (Citrus bigaradia, citrus hystrix, Citrus aurantifolia, citrofurtunella microcarpa, Citrus aurantium, Citrus reticulata), Bitterorange-Extrakt (Schale oder Frucht), Kirsch-Extrakt der spanischen Cherry-Kirsche, Kiwi-Extrakt (Actinidia chinensis), Papayafrucht-Extrakt (Caricae papayae), Tee-Extrakt [Blätter von grünem oder schwarzem Tee, Blätter oder Rinde von New Jersey Tee (Ceanthus velutinas)], Kaffebohnen-Extrakt (INCI-Name: Coffee Bean Extract; von grünen oder gerösteten Bohnen), Prunus-Extrakt (Prunus armeniaca, Prunus dulcis, Prunus persica, Prunus domestica, Prunus spinosa, Prunus serotina, Prunus virginiana), Extrakte der Rinde des mexikanischen Hautbaumes (Mimosa tenuiflora), Angelikawurzel-Extrakt (Angelica archangelica), Pongamia pinnata-Extrakt, Tomatenextrakt. Derartige Pflanzenextrakte sind kommerziell erhältlich, z.B. von DRAGO-CO, Holzminden, Deutschland.

Der Gehalt an diesen Pflanzenextrakten kann zwischen 0 und 40 Gew-% liegen vorzugsweise 0,1 bis 40 Gew-%, insbesondere 1,5 bis 20 Gew-%, wobei auch Gemische dieser Extrakte sowie Gemische mit Acerola-Extrakt in der Wirkstoffzubereitung enthalten sein können.

Der Zusatz der oben genannten Pflanzenextrakte kann den Radikalschutzfaktor je nach Pflanze und entsprechend zugesetzter Menge um ein Mehrfaches erhöhen, wobei vermutlich synergistische Wechselwirkungen auftreten, deren Zusammenhänge noch nicht vollständig geklärt werden konnten.

Es können zusätzlich Antioxidationsmittel eingesetzt werden, wie Vitamine, z.B. Vitamin C und Derivate davon, beispielsweise Ascorbylacetate, -phosphate und -palmitate; Vitamin A und Derivate davon; Folsäure und deren Derivate, Vitamin E und deren Derivate, wie Tocopherylacetat; Flavone oder Flavonoide; Aminosäuren, wie Histidin, Glycin, Tyrosin, Tryptophan und Derivate davon; Carotinoide und Carotine, wie z.B $\alpha$-Carotin, $\beta$-Carotin; Harnsäure und Derivate davon; $\alpha$-Hydroxysäuren wie Citronensäure, Milchsäure, Äpfelsäure; Stilbene und deren Derivat usw.

Vitamine sind auch im Gemisch mit Enzymen als weiterer Anteil in der Wirkstoffzubereitung oder in der kosmetischen Zusammensetzung neben der Wirkstoffzubereitung enthalten. Der Gehalt in der Wirkstoffzubereitung kann wenigstens 0,5 Gew-% eines Gemisches aus Enzymen und Vitaminen betragen, das wenigstens 150 Einheiten/ml (U/ml) superoxiddismutase (SOD) enthält.

Das eingesetzte Gemisch aus Enzymen und Vitaminen ist speziell ein durch Ultraschallbehandlung hergestelltes Aufschlußprodukt einer Hefe, wobei das Aufschlußprodukt SOD, Protease, Vitamin $H_2$, Vitamin $B_6$, Vitamin $B_{12}$, Vitamin $D_2$ und Vitamin E enthält. Vorzugsweise enthält es wenigstens 150 U/ml SOD, Protease und die Vitamine B und D, wobei das Verhältnis SOD:Protease als internationale Einheiten wenigstens im Bereich von 3:1 bis 8:1 liegt.

Besonders vorteilhaft für die Herstellung des Enzym-/Vitamingemisches ist ein Aufschlußverfahren mittels Ultraschall, das in der DE 4241154C1 beschrieben ist und bei dem in einer Ultraschall-Durchflußzelle eine Zelldispersion oder Suspension durch einen Beschallungsraum geführt wird, bei dem die Sonotrode zur Hälfte bis Zweidrittel ihrer Länge in die Durchflußzelle hineinragt und in das zu beschallende Medium eintaucht. Dabei hat die Sonotrode einen Winkel von 80,5 bis 88,5°, und das Verhältnis Eintauchlänge der Sonotrode in mm zum Beschallungsvolumen in ml wird auf einen Wert von 1:1,1 bis 1:20 eingestellt. Der Feststoffanteil in dem zu beschallenden Medium liegt im Bereich von 1:0,02 bis 1:2,2 (Gew.-%).

Als Zelldispersion können Hefen, wie Bäckerhefe, Brauereihefe, Weinhefe sowie besonders behandelte Hefen, wie z.B. SOD-angereicherte Hefen, eingesetzt werden. Eine vorteilhaft einzusetzende Zelldispersion enthält z. B. Saccharomyces cerevisiae.

Der Zusatz von z.B. 1 Gew-% eines solchen Hefeaufschlußproduktes aus Bäckerhefe oder Biohefe als Bestandteil des Zusammenlagerungsgebildes mit Cyclodextrinen kann den an sich schon hohen Radikalschutzfaktor wesentlich erhöhen. Zum Radikalschutzfaktor werden weiter unten noch nähere Ausführungen gemacht.

Als Cyclodextrine können handelsübliche $\alpha$-, $\beta$- oder $\gamma$-Cyclodextrine (Wacker-Chemie) oder Gemische davon eingesetzt werden.

Die Wirkstoffzubereitung kann zusätzlich zu den oben genannten Basiskomponenten auch einen Extrakt von Acerolafrüchten (Malpighia punidifolia) enthalten. Acerola ist ein kleiner in Westindien, im nördlichen Südamerika, in Mittelamerika, Florida und Texas beheimateter Baum und reich an Vitamin C und anderen Wirkstoffen wie Vitamin A, Thiamin, Riboflavin und Niacin, die zusammen mit verschiedenen anderen Bestandteilen, wie Phosphor, Eisen, Calcium eine komplexe Wirkung entfalten können. Der wäßrige Acerola-Extrakt liegt normalerweise als pulverisiertes Produkt vor.

Als weiterer Wirkstoff in der Gesamtzusammensetzung der kosmetischen Zubereitung und zusätzlich zu dem o.g. Wirkstoffkomplex können in einer besonders bevorzugten Ausführungsform enthalten sein ein oder mehrere der folgenden Bestandteile:

(1) Extrakte oder behandelte Extrakte von freie Radikale bindenden oder feuchtigkeitsbindenden Pflanzen, ausgewählt unter Acerolafrüchten (Malpighia punidifolia), Camellia oleifera, Colunsonia canadensis und Hibiscus sabdariffa;

(2) Extrakte oder behandelte Extrakte von freie Radikale bindenden oder feuchtigkeitsbindenden Algen, ausgewählt unter Omegaplankton mit hohem Anteil an Cerebrosid-Stimulantien, Mikro-Algen der Gattung Chlorella und mit Byssus (Muschelseide) assoziierten Makro-Algen der Gattung Ulva als biotechnologische Proteinfraktion und nachfolgender Assoziierung mit Dextrin, wobei das Produkt im Gemisch mit Peptiderivaten vorliegt, die von $\alpha$-MSH abgeleitet und mit Xanthin assoziiert sind;

(3) natürliche und synthetische Polymere, ausgewählt unter Chitosanglycolat, Kondensationsprodukte von Milchpulver und aktivierten Fettsäuren;

(4) hartmagnetische Einkristalle aus Bariumhexaferrit mit einer Koerzitivfeldstärke von 3000-5000 Oe und einer Korngröße von 50-1200 nm eingelagert in oder im Gemisch mit asymmetrischen lamellaren Aggregaten aus Phospholipiden und Fluorcarbonen; sowie

(5) weitere Wirk- und Trägerstoffe, ausgewählt unter Hyaluronsäure, Omega CH Aktivator, Behentrimoniumchlorid, Passionsblumenöl sowie modifizierter Kaolin.

Der genannte modifizierte Kaolin wird gemäß WO96/17588 enthalten und ist mit sphärischen $TiO_2$- oder $SiO_2$-Teilchen mit einer Teilchengröße <5 μm modifiziert, wobei die sphärischen Teilchen einen Anteil an der Kaolinmischung von 0,5 bis 10 Gew-% haben. Das Präparat erhält dadurch ein sehr weiches Hautgefühl und eine zusätzliche entzündungswidrige Wirksamkeit.

Der modifizierte Kaolin kann einen Anteil von 0,1 bis 6 Gew-% haben, bezogen auf die Gesamtmenge des Kosmetikums.

Die genannten hartmagnetischen Teilchen zur Förderung der Durchblutung können solche sein, wie sie in der WO95/03061 beschrieben sind oder solche mit kleineren Teilchengrößen und im Gemisch mit asymmetrischen lamellaren Aggregaten, die bis zum Sättigungsdruck mit Sauerstoff beladen sind, wobei der Anteil der Magnetteilchen, bezogen auf die Gesamtzusammensetzung des Kosmetikums im Bereich von 0,01 bis 10 Gew-% liegen kann.

Die genannten asymmetrischen lamellaren Aggregate sind aus WO94/00098 bekannt und bestehen aus Phospholipiden und mit Sauerstoff beladenem Fluorcarbon oder Fluorcarbongemisch. Der Gehalt an Fluorcarbon liegt im Bereich von 0,2 bis 100 % Gewicht/Volumen, wobei das Phospholipid einen Phosphatidylcholingehalt von mehr als 30 bis 99 Gewichts-% hat, und wobei diese Aggregate eine Hautpenetrierung in Abhängigkeit von der kritischen Löslichkeitstemperatur der Fluorcarbone besitzen.

Die Aggregate können auch zusätzlich allein nur mit Sauerstoff beladen in der kosmetischen Zubereitung vorliegen. Der Anteil kann zwischen 2,5 und 20 Gew-% betragen, bezogen auf die Gesamtzusammensetzung des Kosmetikums.

Diese Aggregate sind Sauerstoffträger und ermöglichen ein Penetrieren des Sauerstoffs in die Haut und damit eine bessere Versorgung der Haut mit Sauerstoff.

Das erfindungsgemäße Präparat enthält weiterhin kosmetische Hilfs- und Trägerstoffe, wie sie üblicherweise in solchen Zubereitungen verwendet werden, z.B. Wasser, Glycerin, Propylenglycol, Konservierungsmittel, Farbstoffe, Pigmente mit färbender Wirkung, Verdickungsmittel, weichmachende Substanzen, feuchthaltende Substanzen, Duftstoffe, Alkohole, Polyole, Elektrolyte, polare und unpolare öle, Polymere, Copolymere, Emulgatoren, Wachse, Stabilisatoren.

Es ist weiterhin vorteilhaft, den erfindungsgemäßen Zusammensetzungen entsprechende wasser- und/oder öllösliche UVA- oder UVB-Filter oder beide zuzusetzen. Zu vorteilhaften öllöslichen UVB-Filtern gehören 4-Aminobenzoesäure-Derivate wie der 4-(Dimethylamino)-benzoesäure-(2-ethylhexyl)ester; Ester der Zimtsäure wie der 4-Methoxyzimtsäure(2-ethylhexyl)ester, Benzophenon-Derivate wie 2-Hydroxy-4-methoxybenzophenon; 3-Benzylidencampher-Derivate wie 3-Benzylidencampher.

Wasserlösliche UVB-Filter sind z.B. Sulfonsäurederivate von Benzophenon oder von 3-Benzylidencampher oder Salze wie das Na- oder K-Salz der 2-Phenylbenzimidazol-5-sulfonsäure.

Zu UVA-Filtern gehören Dibenzoylmethan-Derivate wie 1-Phenyl-4-(4'-isopropylphenyl)propan-1,3-dion.

Bevorzugt als Sonnenschutzfilter sind anorganische Pigmente auf Basis von Metalloxiden, wie $TiO_2$, $SiO_2$, Zno, $Fe_2O_3$, $ZrO_2$, MnO, $Al_2O_3$, die auch im Gemisch untereinander oder mit organischen Filtern eingesetzt werden können.

Besonders bevorzugt als anorganische Pigmente sind agglomerierte Substrate von $TiO_2$ und/oder ZnO mit $SiO_2$ gemäß WO99/06012.

Besonders vorteilhaft einzusetzende $SiO_2$-Teilchen sind hochmonodisperse, unporöse, sphärische $SiO_2$-Teilchen gemäß DE 3616133, die durch hydrolytische Polykondensation von Tetraalkoxysilan in wäßrig-alkoholisch-ammoniakalischen Medium erzeugt werden, wobei ein Sol von Primärteilchen erzeugt wird und anschließend durch ein kontinuierliches, nach Maßgabe des Abreagierens kontrolliertes Zudosieren von Tetraalkoxysilan die erhaltenen $SiO_2$-Teilchen auf die gewünschte Teilchengröße von etwa 0,05 bis 10 $\mu$m bringt.

Pigmente, Pigmentgemische oder Pulver mit pigmentartiger Wirkung, worunter auch solche mit Perlglanz-Effekt zu verstehen sind, können zum Beispiel weiterhin umfassen Glimmer, Kaolin, Talkum, Glimmer-Titanoxid, Glimmer-Titanoxid-Eisenoxid, Wismutoxychlorid, Nylonkügelchen, Keramikkügelchen, expandierte und nichtexpandierte synthetische Polymerpulver, pulverförmige natürliche organische Verbindungen wie gemahlene Festalgen, verkapselte und unverkapselte Getreidestärken sowie Glimmer-Titanoxid-organischer Farbstoff.

Als Erweichungsmittel können normalerweise eine Vielzahl von Verbindungen eingesetzt werden, wie Stearylalkohol, Glycerylmonoricinoleat, Glycerylmonostearat, Propan-1,2-diol, Butan-1,3-diol, Cetylalkohol, Isopropylisostearat, Stearinsäure, Isobutylpalmitat, Oleylalkohol, Isopropyllaurat, Decyloleat, Octadecan-2-ol, Isocetylalkohol, Cetylpalmitat, siliconöle wie Dimethylpolysiloxan, Isopropylmyristat, Isopropylpalmitat, Polyethylenglycol, Lanolin, Kakaobutter, pflanzliche öle wie Maisöl, Baumwollsamenöl, Olivenöl, mineralische öle, Butylmyristat, Palmitinsäure usw.

Kosmetische Zusammensetzungen mit der erfindungsgemäßen Wirkstoffzubereitung können als O/W- oder W/O-Emulsionen vorliegen. Geeignete Emulgatoren für O/W-Emulsionen sind beispielsweise Anlagerungsprodukte von 2-30 Mol Ethylenoxid an lineare $C_8$-$C_{22}$-Fettalkohole, an $C_{12}$-$C_{22}$-Fettsäuren und an $C_8$-$C_{15}$-Alkylphenole; $C_{12}$-$C_{22}$-Fettsäuremono- und -diester von Anlagerungsprodukten von 1-30 Mol Ethylenoxid an Glycerin; Glycerinmono- und -diester sowie Sorbitanmono- und diester von $C_6$-$C_{22}$-Fettsäuren: Polyol- und Polyglycerinester; Anlagerungsprodukte von Ethylenoxid an Ricinusöl; Betaine wie Kokosalkyldimethylammoniumglycinat oder Kokosacylaminoethylhydroxyethylcarboxymethylglycinat (CTFA: Cocamidopropyl Betaine) sowie ampholytische Tenside.

Geeignete Emulgatoren für W/O-Emulsionen sind beispielsweise Anlagerungsprodukte von 2-15 Mol Ethylenoxid an Ricinusöl; Ester von $C_{12}$-$C_{22}$-Fettsäuren

und Glycerin, Polyglycerin, Pentaerythrit, Zuckeralkoholen (z.B. Sorbit), Polyglucosiden (z.B. Cellulose); Polyalkylenglycole; Wollwachsalkohole; Copolymere von Polysiloxan-Polyalkylpolyether.

Der Wassergehalt einer Zubereitung mit dem Wirkstoffkomplex kann in weiten Bereichen schwanken und liegt vorzugsweise bei 5 bis 90 Gew-%, wobei geringe Wassergehalte von ca. 0,5-8 Gew-% insbesondere bei Lippenstiften zu verzeichnen sind.

Die besonders vorteilhafte kosmetische Zubereitung mit der Wirkstoffkomponente Ultraschall-Aufschlußprodukt von Hefen schützt in besonders wirksamer Weise gegen den Angriff freier Radikale auf die Haut sowohl allein als auch in Kombination mit anderen Wirkstoffen, und sie zeigt eine überraschende Wirkung auf die öffnung der Hautporen ähnlich der Wirkungsweise eines Reinigungsmittels (peeling). Dadurch kommen andere Eigenschaften durch weitere Inhaltsstoffe der kosmetischen Zubereitung, wie anhaltende verbesserte Feuchthaltung und Glättung der Haut und somit eine noch mehr verstärkte, langanhaltende Verbesserung des Gesamtzustandes der Haut zur Geltung.

Die erfindungsgemäße kosmetische Wirkstoffzubereitung schützt in besonders wirksamer Weise gegen den Angriff freier Radikale auf die Haut sowohl allein als auch in Kombination mit anderen Wirkstoffen. Sie hat einen hohen Radikalschutzfaktor zwischen 100 und 4000 x $10^{14}$ Radikale/mg.

Der Radikalschutzfaktor (RPF) bestimmt die Aktivität einer Substanz zur Bindung freier Radikale gegenüber einer Testsubstanz. Diese Testsubstanz besteht aus einem sehr reaktionsfähigen, halbstabilen Radikal, das mit allen bekannten Antioxidationsmitteln reagiert. Zu solchen Radikalen gehören Nitroxide, wie Proxo (2,2,5,5-Tetramethyl-1-dihydropyrrolinoxy-nitroxid), Tempol (2,2,6,6-Tetramethyl-1-piperidinoxy-4-ol-nitroxid), DTBN (Di-tert-butyl-nitroxid oder vorzugsweise DPPH (1,1-Diphenyl-2-picryl-hydrazyl).

Die Messung des RPF erfolgt in der Weise, daß die Signalamplitude des Testradikals durch Elektronenspinresonanz (ESR/EPR) vor und nach dem Vermischen mit einem Antioxidationsmittel gemessen und daraus der RPF berechnet wird. Für eine Reihe von Standard-Antioxidationsmitteln ist der RPF bekannt, so liegt er für all-trans-Retinol bei 827, all-trans-Retinolacetat bei 196; für DL-$\alpha$-Tocopherol bei 41200 und für $\alpha$-Tocopherolacetat bei 48, jeweils x $10^{14}$ Radikale/mg.

Die erfindungsgemäße kosmetische Wirkstoffzubereitung, die, wenn sie als Zusammenlagerungsgebilde innerhalb des Gels zwischen dem "Assoziationskomplex" der Bestandteile (a) bis (d) und den Komponenten (f) und (g) vorliegt, hat bei einer Konzentration von (a) und (b) von jeweils 10 Gew-% allein einen RPF von 1255 und liegt damit sehr hoch gegenüber üblichen Wirkstoffen in Kosmetikformulierungen mit deklarierten Radikalfängern, die Werte von etwa 4 bis 40 erreichen. Dabei sind die eigentlichen Wirkstoffe in (a) und (b) nur in einer Konzentration von maximal 2 Gew-% vorhanden. Die Komponente (e) erhöht den Radikalschutzfaktor weiterhin je nach Konzentration um einen bestimmten Wert. Durch Zugabe der Cyclodextrine, die einen Radikalschutzfaktor von 0 haben, wird überraschenderweise eine weitere Steigerung dieses Faktors um das 1,3- bis 10-fache beobachtet.

Unter "hohem Radikalschutzfaktor" wird in der vorliegenden Erfindung ein Wert von 100 oder höher verstanden, vorzugsweise 1000 oder höher. Dieser Wert kann bei bestimmten erfindungsgemäßen Kombinationen von Pflanzenextrakten und dem eigentlichen Assoziationskomplex auf 10000 und höher gesteigert werden. Entsprechende kosmetische Kompositionen mit solchen Zubereitungen haben Radikalschutzfaktoren je nach Anteil der Zubereitung von z.B. 40 bis 400 oder höher.

Das genaue Meßverfahren für den Radikalschutzfaktor ist beschrieben von Herrling, Groth, Fuchs und Zastrow in conference Materials "Modern Challenges To The Cosmetic Formulation" 5.5.-7-5.97, Düsseldorf, S. 150-155, Verlag f. chem. Ind. 1997. Dabei wird, ausgehend von der bekannten Konzentration der Testsubstanz (hier: DPPH) oder der Anzahl von dessen freien Radikalen (Radikale pro ml) eine signalamplitude $S_1$ mittels eines ESR-Spektrometers gemessen. Das Testradikal ist ebenso wie das Antioxidationsmittel in einer (z.B. 0,1 m) Wasser/Alkohollösung gelöst. Dann wird die signalamplitude $S_2$ des Antioxidationsmittels gemessen. Die normalisierte Differenz zwischen den beiden Signalamplituden ist der Reduktionsfaktor RF.

$$RF = (S_1 - S_2) / S_1$$

Das Ergebnis der Radikalreduzierung der Testsubstanz RC x RF wird zu der Menge der Produkteingabe PI (mg/ml) normalisiert. Dabei ist RC die Menge der Testsubstanz, d.h. die bekannte Anzahl der Radikale der Testsubstanz. Der Radikalschutzfaktor wird nach der folgenden Gleichung berechnet

$$RPF = \frac{RC\ [Radikale/ml]\ x\ RF}{PI\ [mg/ml]}$$

Das Ergebnis ist

$$RPF = N\ x\ 10^{14}\ [Radikale\ pro\ mg],$$

wobei N eine positive reale Zahl ist, und der RPF vereinfacht auf den Zahlenwert von N verkürzt werden kann. Diese Verkürzung ist in den Beispielen der vorliegenden Erfindung benutzt.

Der Radikalschutzfaktor kann mittels eines handlichen und sehr einfach konstruierten ESR-Spektrometers (GALENUS GmbH, Berlin, Deutschland) bestimmt werden und ist eine neue Größe zur Kennzeichnung kosmetischer Produkte hinsichtlich ihrer Fähigkeit, freie Radikale zu binden. Das Verfahren ist ein invitro-Verfahren, bei dem keine individuellen Eigenschaften des kosmetischen Anwenders die Antioxidantien beeinflussen.

Weitere vorteilhafte Wirkungen von Produkten mit der erfindungsgemäßen Wirkstoffzubereitung zusammen mit anderen Wirk- und Trägerstoffen sind eine langanhaltende Verbesserung des Gesamtzustandes der Haut, Verzögerung des Alterungsprozesses der Haut, anhaltende verbesserte Feuchthaltung und Glättung der Haut. Die oben beschriebene besonders vorteilhafte Ausführungsform mit einem zusätzlichen Algen-Peptid-Produkt und hartmagnetischen Bariumhexaferrit-Einkristallen zeigt ein besonders Allergie-reduziertes Risiko nach Allergie- und dermatologischer Testung.

Die Verwendung des erfindungsgemäßen kosmetischen Präparates kann z.B. erfolgen in Sonnencremes, Sonnengelen, Aftersun-Produkten, Tagescremes, Nachtcremes, Masken, Körperlotionen, Reinigungsmilch, Make up's, Lippenstiften, Augenkosmetik, Haarmasken, Haarspülungen, Haarshampoos, Duschgelen, Duschölen, Badeölen und weiteren üblichen Produkten. Besonders vorteilhaft liegt die erfindungsgemäße Wirkstoffzubereitung in einer Creme, Lotion, einem Make-up, Fluid, Gel oder Lippenstift vor. Zu vorteilhaften kosmetischen Präparaten gehören auch Zahnpasten und Mundwässer unter dem speziellen Aspekt einer Neutralisierung freier Radikale im Mund von Rauchern, sowie als spezialcreme für Hände und Gesicht von Rauchern. Die Herstellung derartiger Produkte erfolgt auf eine Weise, wie sie dem Fachmann auf diesem Gebiet bekannt ist. Es können bei Wahl entsprechender Trägerstoffe auch entsprechende Pharma-Zubereitungen hergestellt werden.

Die Erfindung soll nachstehend durch Beispiele näher erläutert werden. Alle Angaben erfolgen in Gewichtsprozent, sofern nichts anderes angegeben ist.

Herstellung des Wirkstoffkomplexes

Zur Herstellung der Gelgrundlage wurde Gelpulver, wie Carbomer, in Wasser gegeben, darin homogenisiert und anschließend z.B. mit Triethanolamin neutralisiert. Danach erfolgte eine Zugabe von Ethanol und Glycerin zur besseren Einarbeitbarkeit, wobei die entstehende Mischung gut verrührt wurde. Zu dieser Gelgrundlage wurde ein Gemisch von Phospholipiden (Phoslipon®), Quebracho-Extrakt und Seidenraupenextrakt gegeben und bei einer Temperatur von höchstens 45 °C damit vermischt. Anschließend erfolgte die Zugabe eines weiteren Teiles des obigen Gels oder eines zweiten Gels, wie Guar Propyltriammoniumchlorid, das gut mit dem Gesamtgemisch bei erhöhter Temperatur jedoch unter 45 °C verrührt wurde. Schließlich wurde das Ultraschall-Aufschlußprodukt sowie Cyclodextrin bei etwa 40 °C und unter sorgfältigem Mischen hinzugegeben.

Man erhielt auf diese Weise die erfindungsgemäße Wirkstoffzubereitung, im folgenden als "Komplex" bezeichnet.

Für den Fall, wo die Wirkstoffzubereitung weitere Bestandteile, wie Acerola-Extrakt oder Extrakte von Tee, Kaffee, Kiwi, citrus, Kirsche, Papaya, Tomate, Pongamia pinnata oder Hautbaum enthielt, wurde dieser Extrakt der Phospholipidmischung zugesetzt und dann mit dem Gel vermischt (erweiterter Wirkstoffkomplex).

Beispiel 1 **Tagescreme**

**Phase A** : Carbomer 0,2; Glycerin 2,0; Propylenglycol 1,0;
dest. Wasser q.s. ad 100;
**Phase B** : $C_{12}$-$C_{15}$-Alkyl Cetylalkohol 3,7; Stearath 0,5; Jojobaöl 1,0;
**Phase C** : Triethanolamin 0,2;
**Phase D** : Wirkstoffkomplex mit (a)-(g) 3,5; Konservierungsmittel 0,3.

Die Phasen A und B wurden unter Rühren auf 65 ±2 C° erwärmt und die Phase B in der Phase A homogenisiert. Danach erfolgte die Zugabe der Phase C und entsprechendes Homogenisieren. Dann wurde unter Rühren auf etwa 35 °C abgekühlt und die Phase D unter sorgfältigem Vermischen zugesetzt. Der Wirkstoffkomplex enthielt 1,0 % aus Bäckerhefe und nach dem Ultraschallverfahren der DE 4241154C1 gewonnenes SOD-haltiges Enmzym/Vitaminprodukt.

Der zugesetzte Wirkstoffkomplex enthielt 1% trockenes Gel, 7% Phospholipide, 2% Quebracho-Extrakt, 1% Seidenraupen-Extrakt, 1 % SOD aus Bäckerhefeaufschlußprodukt, 2,5 % β-Cyclodextrine.

Der Radikalschutzfaktor des Wirkstoffkomplexes betrug 12215, und in der Formulierung lag er bei 470.

Vergleichsbeispiel 1

Es wurde eine kosmetische Zusammensetzung wie in Beispiel 1 hergestellt, wobei in dem Wirkstoffkomplex kein Dextrin enthalten war. Der Radikalschutzfaktor des Wirkstoffkomplexes betrug 1925, der RPF in der Formulierung lag bei 49.

Beispiel 2

Erweiterte Wirkstoffzubereitung

**Phase A**: Phospholipid 5,0; Glycerin 8,0; Ethanol 7,0; dest. Wasser 2,0;
**Phase B**: Pongamia, pinnata 2,0; Tomatenextrakt 5,0; Grüner Tee-Extrakt 2,0; Coffee-Extrakt 1,0;

**Phase C**: Konservierungsmittel 0,2; dest. Wasser ad 100;

**Phase D**: Carbomer 2,0; Triethanolamin 2,0;

**Phase E**: Wirkstoffkomplex mit (a) bis (g) 5,0 (Zusammensetzung wie Beispiel 1, außer 3,4 % β-Cyclodextrin und 1,5 % Ultraschall-Biohefe-Aufschlußprodukt).

Die Phase A wurde unter Mischen (500-1000 U/Min) und Homogenisieren (10000-30000 U/Min) der Einzelkomponenten bei etwa 40 °C hergestellt. Die Phase B wurde unter Mischen (600 U/Min) und Homogenisieren (30000 U/Min) der Einzelkomponenten bei <40 °C hergestellt. Danach wurde Phase C bei ebenfalls <40 °C zugesetzt und dann homogenisiert (30000 U/Min), anschließend erfolgte die Zugabe der Phase D und Rühren. Schließlich wurde nach Abkühlen auf etwa 35 °C die Phase E unter sorgfältigem Vermischen zugesetzt. Die Phase E war ähnlich wie im Beispiel 1 erhalten worden. Der RPF des Wirkstoffkomplexes (Phase E) betrug 7380, der RPF in der erweiterten Wirkstofformulierung (Phasen A+B+C+D+E) lag bei 9870.

Vergleichsbeispiel 2

Es wurde eine kosmetische Zusammensetzung wie in Beispiel 2 hergestellt, wobei in dem Wirkstoffkomplex kein Dextrin enthalten war. Der RPF des Wirkstoffkomplexes betrug 7380, der RPF in der erweiterten Wirkstofformulierung lag bei 7810.

**Patentansprüche**

1. Kosmetische Wirkstoffzubereitung mit synergistisch erhöhtem Radikalschutzfaktor, **gekennzeichnet durch** einen Gehalt an

   (a) einem **durch** Extraktion der Rinde von Quebracho blanco und nachfolgender enzymatischer Hydrolyse gewonnenem Produkt, das wenigstens 90 Gew-% Proanthocyanidin-Oligomere und höchstens 10 Gew-% Gallussäure enthält, wobei der Gehalt von (a), das in einer an Mikrokapseln gebundenen Wirkstoffkonzentration von 2 Gew-% vorliegt, im Bereich von 0,1 bis 10 Gew-% liegt;
   (b) einem **durch** Extraktion gewonnenen Seidenraupenextrakt, der das Peptid Cecropine, Aminosäuren und ein Vitamingemisch enthält, wobei der Gehalt von (b) im Bereich von 0,1 bis 10 Gew-% liegt;
   (c) einem nichtionischen, kationischen oder anionischen Hydro-Gel oder Gemisch von Hydro-Gelen mit einem Gehalt von (c) im Bereich von 0,1 bis 5 Gew-%;
   (d) einem oder mehreren Phospholipiden mit einem Anteil von 0,1 bis 30 Gew-%;

   (e) einem wenigstens 150 Einheiten Superoxiddismutase pro ml enthaltenden Ultraschall-Aufschlußprodukt einer Hefe, wobei der Gehalt des Aufschlußproduktes im Bereich von 0,5 bis 4 Gew-% liegt;
   (f) einem oder mehreren Cyclodextrinen, ausgewählt unter α-, β- und γ-Cyclodextrinen, mit einem Gehalt von 0,5 bis 8 Gew-%; und
   (g) dem Rest Wasser;

   jeweils bezogen auf das Gesamtgewicht der Wirkstoffzubereitung.

2. Zubereitung nach Anspruch 1, **dadurch gekennzeichnet, daß** sie zusätzlich einen Extrakt von Acerolafrüchten Malpighia punidifolia mit einem Gehalt im Bereich von 1 bis 30 Gew-%, bezogen auf das Gesamtgewicht der Wirkstoffzubereitung.

3. Zubereitung nach Anspruch 1, **dadurch gekennzeichnet, daß** der Anteil der Bestandteile in folgenden Bereichen liegt:

   Wirkstoffkapseln gemäß (a) und (b) im Bereich von 0,5 bis 3 Gew-%, Hydro-Gel gemäß (c) im Bereich von 0,1 bis 3 Gew-%, Cyclodextrin und Hefeaufschlußprodukt jeweils im Bereich von 1 bis 3 Gew-%.

4. Zubereitung nach Anspruch 1, **gekennzeichnet durch** einen Radikalschutzfaktor im Bereich von 100 bis 3500, gemessen **durch** Bestimmung der Anzahl freier Radikale einer Lösung einer Testsubstanz $(S_1)$ mittels Elektronenspinresonanz (ESR) im Vergleich mit dem ESR-Meßergebnis der kosmetischen Wirkstoffzubereitung nach der Beziehung RPF = (RC x RF) / PI
   worin RF = $(S_1-S_2)$ / $S_1$ ; RC = Konzentration der Testsubstanz (Radikale/ml); PI = Konzentration der Wirkstoffzubereitung (mg/ml).

5. Zubereitung nach Anspruch 1, **dadurch gekennzeichnet, daß** der Extrakt (a) wenigstens 99 Gew-% Proanthocyanidin-Oligomere und höchstens 1 Gew-% Gallussäure enthält.

6. Zubereitung nach Anspruch 1, **dadurch gekennzeichnet, daß** die in (b) enthaltenen Aminosäuren Aspartinsäure, Asparagin, Threonin, Serin, Glutaminsäure, Prolin, Glycin, Alanin, Valin, Cystein, Methionin, Isoleucin, Leucin, Tyrosin, Phenylalanin, Lysin, Histidin, Arginin umfassen.

7. Zubereitung nach Anspruch 1, **dadurch gekennzeichnet, daß** die Bestandteile (a) und (b) in Konzentrationen von jeweils 0,1 bis 3 Gew-% in der Wirkstoffzubereitung vorliegen.

8. Zubereitung nach Anspruch 1, **dadurch gekennzeichnet, daß** die Wirkstoffzubereitung in einer kosmetischen Zusammensetzung vorliegt, die weiterhin noch einen oder mehrere der folgenden Bestandteile enthält:

   (1) Extrakte oder behandelte Extrakte von freie Radikale bindenden oder feuchtigkeitsbindenden

   (1.1) Pflanzen, ausgewählt unter Acerolafrüchten (Malpighia punidifolia), Camellia oleifera, Colunsonia canadensis und Hibiscus sabdariffa, oder
   (1.2) Algen, ausgewählt unter Omegaplankton mit hohem Anteil an Cerebrosid-Stimulantien, Mikro-Algen der Gattung Chlorella und mit Byssus (Muschelseide) assoziierten Makro-Algen der Gattung Ulva als biotechnologische Proteinfraktion und nachfolgender Assoziierung mit Dextrin, wobei das Produkt im Gemisch mit Peptiderivaten vorliegt, die von $\alpha$-MSH abgeleitet und mit Xanthin assoziiert sind ;

   (2) Hefeaufschlußprodukte, ausgewählt unter Bäckerhefe, Brauereihefe und Weinhefe und hergestellt nach einer schonenden Ultraschallbehandlung der wäßrigen Hefen;
   (3) natürliche und synthetische Polymere, ausgewählt unter Chitosanglycolat, Kondensationsprodukte von Milchpulver und aktivierten Fettsäuren;
   (4) hartmagnetische Einkristalle aus Bariumhexaferrit mit einer Koerzitivfeldstärke von 3000-5000 oe und einer Korngröße von 50-1200 nm eingelagert in oder im Gemisch mit asymmetrischen lamellaren Aggregaten aus Phospholipiden und Fluorcarbonen; und
   (5) weitere Wirkstoffe, ausgewählt unter Chitosanglycolat, Hyaluronsäure, Omega CH Aktivator, Behentrimoniumchlorid, Passionsblumenöl, sowie Trägerstoffe;
   (6) Gemischen davon.

9. Zubereitung nach Anspruch 1, **dadurch gekennzeichnet, daß** sie zusätzlich einen Gehalt von 0,1 bis 20 Gew-% Pflanzenextrakte aufweist, ausgewählt aus der Gruppe, bestehend aus Citrusschalen- oder -blatt-Extrakte (Citrus bigaradia, Citrus hystrix, Citrus aurantifolia, citrofurtunella microcarpa, Citrus aurantium, Citrus reticulata), Bitterorange-Extrakt (Schale oder Frucht), Kirsch-Extrakt der spanischen Cherry-Kirsche, Kiwi-Extrakt (Actinidia chinensis), Papayafrucht-Extrakt (Caricae papayae), Tee-Extrakt [Blätter von grünem oder schwarzem Tee, Blätter oder Rinde von New Jersey Tee (Ceanthus velutinas)], Kaffebohnen-Extrakt (INCI-Name: Coffee Bean Extract; von grünen oder gerösteten Bohnen), Prunus-Extrakt (Prunus armeniaca, Prunus dulcis, Prunus persica, Prunus domestica, Prunus spinosa, Prunus serotina, Prunus virginiana), Extrakte der Rinde des mexikanischen Hautbaumes (Mimosa tenuiflora), Angelikawurzel-Extrakt (Angelica archangelica) , Pongamia pinnata-Extrakt, Tomatenextrakt und dem restlichen Anteil Trägerstoffe oder weitere Wirkstoffe und Trägerstoffe.

10. Verwendung der kosmetischen Wirkstoffzubereitung mit hohem Radikalschutzfaktor nach einem der Ansprüche 1 bis 9 zusammen mit anderen kosmetischen Stoffen, wie weiteren Wirkstoffen, Hilfsstoffen und Trägerstoffen in kosmetischen Zubereitungen wie Cremes, Gelen, Lotionen, Masken, Make-up's, Shampoos, Stiften, Ölen, Mascara, entsprechenden Sonnenschutzpräparaten sowie Zahnpasten und Mundwässern.

## Claims

1. Cosmetic active substance preparation with a high radical protection factor, which comprises a content of

   a) a product obtained by extraction of the bark of Quebracho blanco and subsequent enzymatic hydrolysis, containing at least 90 percent by weight of proanthocyanidine oligomers and up to 10 percent by weight of gallic acid, where the content of (a), which is available in a concentration of 2 percent by weight linked to a microcapsule ranges from 0.1 to 10 wt.%;
   (b) an extract of the silkworm obtained by extraction, containing the peptide cecropine, amino acids and a vitamin mix, where the content of (b) ranges from 0.1 to 10 wt.%;
   (c) a non-ionic, cationic or anionic hydrogel or mixture of hydrogels, where the content of (c) ranges from 0.1 to 5 wt%;
   (d) one or several phospholipids in the range of 0.1 up to 30 wt.%;
   (e) an ultrasound decomposition product of a yeast containing at least 150 units of superoxide dismutase per ml, wherein the content of the decomposition product is in the range from 0.5 to 4 percent by weight;
   (f) one or more cyclodextrines selected from the group consisting of $\beta$- and $\gamma$-cyclodextrins with a share of 0.5 to 8 % by weight; and
   (g) up to 100 percent by weight of water;

   related to the total weight of the active substance preparation each.

2. Preparation according to claim 1, **characterised by** additionally comprising an extract of acerola fruits Malpighia punidifolia, wherein the content (g) is in the range from 1 to 30 wt.%; related to the total weight of the active substance preparation.

3. Preparation according to claim 1, wherein the portions of the components lie within the following ranges:

active substance capsules according to (a) and (b) ranging from 0.5 to 3 wt.%, hydro gel according to (c) ranging from 0.1 to 3 wt.%, cyclodextrin and yeast decomposition product each in the range of 1 to 3 % by weight.

4. Preparation according to claim 1, wherein the radical protection factor is in the range from 100 to 3500, measured by determining the number of free radicals of a solution of a test substance ($S_1$) by electron spin resonance (ESR) as compared with the ESR measurement result of the cosmetic active substance preparation according to the relationship RPF = (RC x RF) / PI, where RF = ($S_1$-$S_2$) / $S_1$ ; RC = concentration of the test substance (radicals per ml); PI = concentration of the active substance preparation (mg per ml).

5. Preparation according to claim 1, wherein the extract (a) contains at least 99 wt.% of proanthocyanidine oligomers and up to 1 wt.% of gallic acid.

6. Preparation according to claim 1, wherein the amino acids contained in (b) comprise aspertine acid, asparagine, threonine, serine, glutamic acid, proline, glycine, alanine, valine, cysteine, methionine, isoleucine, leucine, tyrosine, phenylalanine, lysine, histidine, arginine.

7. Preparation according to claim 1, wherein the active substance preparation contains portions of the components (a) and (b) each in the range of 0.1 to 3 % by weight.

8. Preparation according to claim 1, wherein the active substance preparation exists as cosmetic composition, further comprising one or several of the following components:

(1) extracts or treated extracts binding free radicals or moisture of

(1.1) plants selected among acerola fruits (Malpighia punidifolia), Camellia oleifera, Colunsonia canadensis and Hibiscus sabdariffa; or
(1.2) algae selected among omega plankton, providing a high portion of cerebrosid stimulants, microalgae of the chlorella species and macro algae of the ulva species associated with byssus (mussel silk) as biotechnological protein fraction and subsequently associated with dextrine, wherein the product appears in the mixture with peptide derivates derived from α-MSH and associated with xanthin;

(2) yeast decomposition products selected among
baker□s yeast, brewer□s yeast, wine yeast and made according to a non-harming ultrasound treatment of the aqueous yeasts;
(3) natural and synthetic polymers selected among chitosanglycolate, condensed products of desiccated milk, and activated fatty acids;
(4) magnetically hard single crystals of bariumhexaferrite having a coercive field intensity of 3000 □ 5000 Oe and a grain size of 50-1200 nm intercalated in or mixed with asymmetric lamellar aggregates of phospholipids and fluorocarbons ; and
(5) other active substances selected among chitosanglycolate, hyaluronic acid, omega CH activator, behentrimonium chloride, passion flower oil and carrier substances;
(6) mixtures thereof.

9. Preparation according to claim 1, wherein it comprises an additional portion of 0.1 to 20 wt.% of plant extracts selected from the group consisting of citrus peel or leaf extracts (Citrus bigaradia, Citrus hystrix, Citrus aurantifolia, Citrofurtunella microcarpa, Citrus aurantium, Citrus reticulata), petitgrain extract (peel or fruit), extract of the Spanish cherry, kiwi extract (Actinidia chinensis), papaya fruit extract (Caricae papayae), tea extract [leaves of green or black tea, leaves or bark of new jersey tea (Ceanthus velutinas)], coffee bean extract (INCI name: coffee bean extract; of green or roasted beans), prunus extract (Prunus armeniaca, Prunus dulcis, Prunus persica, Prunus domestica, Prunus spinosa, Prunus serotina, Prunus virginiana), extracts of the bark of the Mexican skin tree (Mimosa tenuiflora), angelica root extract (Angelica archangelica), Pongamia pinnata extract, tomato extract and the remaining portion of carrier substances or other active substances and carrier substances.

10. Use of the cosmetic substance preparation with high radical protection factor according to each of claims 1 through 9 in combination with other cosmetic substances such as other active substances, auxiliary substances and carrier substances in cosmetic preparations such as creams, gels, lotions, masks, makeup's, shampoos, sticks, oils, mascara, corresponding sun protection preparations as well as tooth paste and mouthwashes.

**Revendications**

1. Préparation cosmétique de principes actifs ayant un facteur de protection contre les radicaux libres élevé, **caractérisée par** une teneur en

(a) un produit obtenu par extraction de l'écorce de *Quebraco blanco* puis hydrolyse enzymatique subséquente du produit obtenu, qui contient au moins 90 % en poids d'oligomère de proanthocyanidine et au plus 10 % en poids d'acide gallique, la teneur en (a), qui est présent en une concentration de principe actif lié à des microcapsules de 2 % en poids, étant de 0,1 à 10 % en poids ;
(b) un extrait de ver à soie obtenu par extraction, qui contient le peptide cecropine, des acides aminés et un mélange de vitamines, la teneur en (b) étant de 0,1 à 10% en poids ;
(c) un hydrogel non ionique, cationique ou anionique ou un mélange d'hydrogels avec une teneur en (c) de 0,1 à 5 % en poids ;
(d) un ou plusieurs phospholipides avec une part de 0,1 à 30 % en poids ;
(e) un produit digesté par ultrasons d'une levure contenant au moins 150 unités de superoxyde dismutase par ml, la teneur en produit digesté étant de 0,5 à 4 % en poids ;
(f) une ou plusieurs cyclodextrines, choisies parmi les cyclodextrines α-, β- et γ-, avec une teneur de 0,5. à 8 % en poids ; et
(g) le reste d'eau ;

en fonction du poids total de la préparation de principes actifs.

2. Préparation selon la revendication 1, **caractérisée en ce qu'**elle contient en outre un extrait du fruit de l'acérola *Malpighia punidifolia* avec une teneur de 1 à 30 % en poids, en fonction du poids total de la préparation de principes actifs.

3. Préparation selon la revendication 1, **caractérisée en ce que** la part des composants est comprise dans les domaines suivants : capsules de principe actif selon (a) et (b) dans un domaine de 0,5 à 3 % en poids, hydrogel selon (c) dans un domaine de 0,1 à 3 % en poids, cyclodextrine et produit digesté de levure dans un domaine de 1 à 3 % en poids chacun.

4. Préparation selon la revendication 1, **caractérisée par** un facteur de protection contre les radicaux libres dans un domaine de 100 à 3 500, mesuré par évaluation du nombre de radicaux libres d'une solution d'une substance de test ($S_1$) par spin-résonance électronique (SRE) en comparaison avec le résultat de mesure SRE de la préparation cosmétique de principes actifs selon la relation FPR = (RC x FR) / AP où FR = ($S_1$-$S_2$)/$S_1$ ; RC = concentration de la substance de test (radicaux/ml) ; AP = concentration de la préparation de principes actifs (mg/ml).

5. Préparation selon la revendication 1, **caractérisée en ce que** l'extrait (a) contient au moins 99 % en poids d'oligomère de proanthocyanidine et au plus 1 % en poids d'acide gallique.

6. Préparation selon la revendication 1, **caractérisée en ce que** les acides aminés contenus dans (b) comprennent l'acide aspartique, l'asparagine, la thréonine, la sérine, l'acide glutamique, la proline, la glycine, l'alanine, la valine, la cystéine, la méthionine, l'isoleucine, la leucine, la tyrosine, la phénylalanine, la lysine, l'histidine, l'arginine.

7. Préparation selon la revendication 1, **caractérisée en ce que** les composants (a) et (b) sont présents à des concentrations de 0,1 à 3 % en poids chacun de la préparation de principes actifs.

8. Préparation selon la revendication 1, **caractérisée en ce que** la préparation de principes actifs est présente dans une composition cosmétique qui contient en outre un ou plusieurs des composants suivants :

(1) des extraits ou des extraits traités de végétaux piégeurs de radicaux libres ou fixant l'humidité

(1.1) des végétaux choisis parmi les fruits de l'acérola (*Malpighia punidifolia*), *Camellia oleifera*, *Colunsonia canadensis* et *Hibiscus sabdariffa ;* ou
(1.2) des algues choisies parmi le plancton oméga à forte teneur en stimulants du cérébroside, les micro algues du genre *Chlorella* et des macro algues associées au byssus du genre *Ulva* comme fraction protéique biotechnologique et ensuite association à la dextrine, le produit étant présent en mélange avec des dérivés peptidiques dérivés de l'α-MSH et associés à la xanthine ;

(2) des produits digestés de levure, choisis parmi la levure de boulanger, la levure de brasserie et la lie de vin et fabriqués par un traitement aux ultrasons des levures aqueuses ;
(3) des polymères naturels et synthétiques, choisis parmi le glycolate de chitosan, les produits de condensation de la poudre de lait et d'acides gras activés ;

(4) des monocristaux magnétiques d'hexaferrite de baryum avec une force de champ coercitif de 3 000 à 5 000 Oe et une taille de grains de 50 à 1 200 nm appliqués sur ou mélangés avec des agrégats lamellaires asymétriques de phospholipides et de fluorocarbones ; et

(5) d'autres principes actifs choisis parmi le glycolate de chitosan, l'acide hyaluronique, l'activateur oméga CH, le chlorure de behentrimonium, l'huile de fleur de la passion, ainsi que des substances porteuses ;

(6) leurs mélanges.

9. Préparation selon la revendication 1, **caractérisée en ce qu'**elle présente en outre une teneur de 0,1 à 20 % en poids d'extraits végétaux, choisis dans le groupe comprenant l'extrait d'écorce ou de feuille de citron (*citrus bigaradia, Citrus hystrix, Citrus aurantifolia*, *Cirofurtunella microcarpa, Citrus aurantium, Citrus reticulata*) , l'extrait d'orange amère (écore ou fruit), l'extrait de cerise cherry, l'extrait de kiwi (*Actinidia chinensis*), l'extrait de papaye (*Caricae papyae*), l'extrait de thé (feuilles de thé vert ou de thé noir, feuilles ou écorce de thé de New Jersey (*Ceanthys velutinas*)], l'extrait de grain de café (Nom INCI : coffee bean extract ; de grains verts ou torréfiés), l'extrait de prune *(Prunus, armeniaca, Prunus dulcis, Prunus persica, Prunus domestica, Prunus spinosa*, *Prunus serotina, Prunus virginiana),* l'extrait d'écorce de bourdaine (*Mimosa tenuiflora*), l'extrait de racine d'Angélique (*Angelica archangelica*), l'extrait de Pongamia pinnata, l'extrait de tomate et le reste étant des substances porteuses ou d'autres principes actifs et substances porteuses.

10. Utilisation de la préparation de principes actifs ayant un facteur de protection contre les radicaux libres élevé selon l'une des revendications 1 à 9 avec d'autres substances cosmétiques, comme d'autres principes actifs, des additifs et des substances porteuses, dans des préparations cosmétiques comme des crèmes, des gels, des lotions, des masques, des maquillages, des shampooings, des rouges à lèvres, des huiles, du mascara, des préparations de protection solaire ainsi que des pâtes dentifrices et des bains de bouche.